# EUROPÄISCHE PATENTSCHRIFT

(11) **EP 0 614 350 B1**
(45) Veröffentlichungstag und Bekanntmachung des Hinweises auf die Patenterteilung: **07.05.1997**
(21) Anmeldenummer: 93919056.7
(22) Anmeldetag: 06.08.1993
(51) Int. Cl.: A61K 7/06, A61K 7/09

(54) **HAARBEHANDLUNGSMITTEL UND VERFAHREN ZUR ANWENDUNG**
HAIR TREATMENT AGENT AND PROCESS FOR ITS USE
AGENT DE SOIN CAPILLAIRE ET PROCEDE D'UTILISATION

(30) Priorität: 01.10.1992 DE 4232944
(43) Veröffentlichungstag der Anmeldung: 14.09.1994
(73) Patentinhaber: Wella Aktiengesellschaft, 64274 Darmstadt (DE)
(72) Erfinder: HOCH, Dieter, D-6102 Pfungstadt-Eich (DE); GROSS, Paul, D-6100 Darmstadt (DE); FLEMMING, Ernst, D-6056 Heusenstamm (DE)
(86) Internationale Anmeldenummer: EP9302095
(87) Internationale Veröffentlichungsnummer: WO9407457

(56) Entgegenhaltungen:
- EP-A- 0 260 641
- EP-A- 0 463 780
- WO-A-89/04164
- FR-A- 2 222 996
- US-A- 4 897 262
- US-A- 5 034 218
- PATENT ABSTRACTS OF JAPAN vol. 4, no. 166 (C-31)(648) 18. November 1980 & JP,A,55 108 811 (LION YUSHI K.K.) 21. August 1980

## Beschreibung

Gegenstand der Erfindung ist ein gelförmiges Mittel zur Behandlung der Haare auf wäßrig-alkoholischer Basis, dadurch gekennzeichnet, daß es ein hochmolekulares Polyacrylsäurehomopolymer und/oder ein mit Decadien vernetztes Methylvinylether-Maleinsaureanhydrid-Copolymer, ein nicht-flüchtiges Polydimethylsiloxan und ein nicht-ionisches und/oder anionisches Tensid enthält, sowie ein Verfahren zur Anwendung des Mittels.

Die Haare werden durch Einwirkungen verschiedener Art in ihren physikalischen, chemischen und morphologischen Eigenschaften negativ beeinflußt. So wird das Haar durch kosmetische Behandlungen, wie wiederholtes Bleichen, Dauerwellen und Färben, aber auch durch häufiges Waschen der Haare mit entfettenden Tensiden, durch Klimaeinflüsse wie Feuchte- und Temperaturunterschiede oder die intensive Einwirkung von Sonnenlicht sowie durch mechanische Behandlung wie Bürsten, Kämmen und Frottieren, insbesondere im Bereich der Haarspitzen stark strapaziert und geschädigt, während das Haar am Haaransatz eine gesunde ungeschädigte Struktur besitzt. Die meist unterschiedliche Haarstruktur zwischen Haaransatz und den Haarspitzen stellt bei der Dauervellbehandlung der Haare ein großes Problem dar. Wird das Dauerwellmittel in seiner Wellwirksamkeit den strapazierten und geschädigten Haarspitzen angepaßt, so erhält man eine nur ungenügende Dauerverformung des Haares am Haaransatz. Paßt man dagegen das Dauerwellmittel in seiner Wellwirksamkeit der gesunden, ungeschädigten Struktur des Haares am Haaransatz an, so kann das Haar an der strapazierten Haarspitze so sehr geschädigt werden, daß es zu einer stark eingeschränkten Lockigkeit bis hin zum Abbrechen der Haare kommen kann.

Bei der Dauerwellbehandlung der Haare mit keratinreduzierenden Verformungsmitteln wird im allgemeinen so verfahren, daß man die zuvor gewaschenen und mit einem Handtuch abfrottierten Haare mit einem Teil des Verformungsmittels durchfeuchtet, sodann in Strähnen aufteilt, diese Strähnen einzeln auf Dauerwellwickler wickelt und anschließend mit dem restlichen Verformungsmittel befeuchtet. Nach Beendigung des Verformungsvorganges werden die Haare mit Wasser gespült, sodann oxidativ fixiert, anschließend die Wickler entfernt, die Haare erneut mit Wasser gespült und gegebenenfalls mit einem Kurpräparat nachbehandelt.

Diese Verfahrensweise besitzt jedoch einige Nachteile. So kann eine derartige Verfahrensweise zu Schädigungen an den Händen des die Verformungsbehandlung ausführenden Friseurs führen (zum Beispiel Allergien oder anderen Hauterkrankungen), da die Hände über die gesamte für das Wickeln erforderliche, etwa 20 Minuten betragende, Zeitspanne mit dem Verformungsmittel in Kontakt kommen. Außerdem ist das vorstehend beschriebene Verfahren nicht sehr haarschonend, da die durch das Vorfeuchten der Haare mit dem Verformungsmittel eintretende Haarerveichung beim Wickelvorgang sehr leicht zu einer Überdehnung des Haares und als Folge hiervon zu Haarbruch und Haarausfall führen kann.

Zudem sind die Haare nach einer Dauerwellverformungsbehandlung meist stark strapaziert, so daß eine Nachbehandlung mit einem Haarkurmittel erforderlich ist, um den Haar wieder einen natürlichen Griff und Glanz zu verleihen.

Aus der EP-B 0 014 479 ist ein Mittel zur Behandlung von Haaren vor dem Wickeln auf Dauerwellwickler bekannt, welches einen Gehalt an einem Methylpolysiloxan und/oder Paraffin bzw. Isoparaffin, einer kationaktiven Verbindung und einer ampholytischen Verbindung aufweist. Dieses Mittel ist jedoch in Bezug auf den Schutz der strapazierten Haarspitzen während des Dauerwellvorganges und somit auf die Gleichmäßigkeit der Dauerwellung nicht voll befriedigend.

Aus der EP-A 0 260 641 ist ein emulsionsförmiges Haarbehandlungsmittel mit haarfestigenden Eigenschaften mit einem Gehalt an einer haarfestigenden Polymerkombination, einem Silikonöl, einem nicht-ionischen Tensid und Ethanol bekannt, welches einen gleichmäßigen Film auf dem Haar hinterläßt und gute Festigungseigenschaften aufweist. Ein Hinweis auf den alleinigen Einsatz von bestimmten Acrylsäurehomopolymeren, welche üblicherweise keine haarfestigenden Eigenschaften aufweisen, ist der EP-A 0 260 641 nicht zu entnehmen. In der US-A 4 954 335 sind klare, nicht-emulsionsförmige Haarkonditionierungsmittel beschrieben, die unter anderem eine Kombination aus nicht-ionischem Tensid, Ethanol sowie eines flüchtigen Silikonöls enthalten.

Den Mitteln können Verdicker in Form von Polyacrylsäurederivaten zugesetzt werden, sie enthalten jedoch zwingend quaternäre Ammoniumverbindungen und eine Amidoaminverbindung mit konditionierender Wirkung. Aus der PCT/WO 89/04164 ist ein Haarkonditionierungsmittel mit einem Gehalt an Verdickungsmittel, eines nicht-flüchtigen Polydiorganosiloxans in Form einer Öl-in-Wasser-Emulsion, und eines nicht-ionischen Tensids bekannt.

Die EP-A 0 463 780 beschreibt ein stabiles Perlglanzshampoo mit einem Gehalt an Silikonölen, das vor der Weiterbehandlung des Haares, beispielsweise mit Dauerwellmitteln, ausgespült wird. Aus der FR-A 2 222 996 ist weiterhin ein Aerosollack mit einem Gehalt an bestimmten Vinylethercopolymeren, nichtionischen Tensiden und Silikonölen bekannt. Eine pflegende und schützende Wirkung der beschriebenen Mittel während einer Dauerwellbehandlung ist nicht bekannt.

Es bestand daher die Aufgabe, ein Mittel zur Behandlung der Haare zur Verfügung zu stellen, welches das Haar, insbesondere die Haarspitzen, während des Dauerwellvorganges schützt und so eine gleichmäßige Verformung der Haare ermöglicht. Zudem soll das Mittel gute glanzgebende Eigenschaften besitzen ohne das Haar zu belasten, gleich, ob es vor oder nach einem Dauerwellvorgang angewandt wird.

Es wurde nunmehr gefunden, daß durch ein gelförmiges Dauerwellvorbehandlungsmittel, dadurch gekennzeichnet, daß es
(A) neben der Komponente (B) als einziges weiteres Polymer 0,3 bis 4 Gewichtsprozent mindestens eines hochmolekularen Acrylsäurehomopolymers mit einem Molekulargewicht von 1.000.000 bis 4.000.000,
(B) 15 bis 30 Gewichtsprozent mindestens eines nichtflüchtigen Polydimethylsiloxans,
(C) 0,1 bis 0,5 Gewichtsprozent mindestens eines nicht-ionischen und/oder anionischen Tensids und
(D) 10 bis 40 Gewichtsprozent Ethanol und/oder Isopropanol
enthält, die gestellte Aufgabe in hervorragender Weise erfüllt wird.

Das Haarbehandlungsmittel enthält die Komponente (A) bevorzugt in einer Menge von 0,4 bis 2 Gewichtsprozent.

Geeignete Acrylsäurehomopolymere sind beispielsweise solche mit einem Molekulargewicht von ca. 1.250.000 (z.B. das Handelsprodukt Acrisint 410 der Firma Sigma, Bergamo, Italien), 3.000.000 (z.B. die Handelsprodukte Carbopol 934, Carbopol 2984 und Carbopol 5984 der Firma B.F. Goodrich, Cleveland, USA oder Acrisint 430 der Firma Sigma) oder 4.000.000 (z.B. die Handelsprodukte Carbopol 940 der Firma B.F. Goodrich and Acrisint 400 der Firma Sigma).

Zur Neutralisation der in dem Haarpflegemittel enthaltenen Polymere können beispielsweise verdünnte Lösungen von Natriumhydroxid, Ammoniak, Triethanolamin oder Diisopropanolamin verwendet werden.

Das erfindungsgemäße Mittel weist bevorzugt einen pH-Wert von 6,5 bis 7,5 auf.

Beispiele für als Komponente (B) geeignete nichtflüchtige Polydimethylsiloxane sind Polydimethylsiloxane mit einer Viskosität von 50 bis 1.000 mm2.s-1, zum Beispiel die Handelsprodukte Abil 500, Abil 450 und Abil 350 der Firma Goldschmidt, Essen, Deutschland, oder Silikon AK 350, Silikon AK 500 und Silikon AK 1000 der Firma Wacker, München, Deutschland, sowie Dow Corning Fluid-200, Dow Corning Fluid-500 und Dow Corning Fluid-350 der Firma Dow Corning, Midland, USA.

Das nicht-ionische Tensid der Komponente (C) ist bevorzugt ausgewählt aus oxethyliertem Rizinusöl, oxethyliertem hydrierten Rizinusöl oder Polyoxyethylensorbitanmonofettsäureester. Das, bevorzugt mit 30 bis 40 Mol Ethylenoxid, oxethylierte Rizinusöl kann beispielsweise in Form der Handelsprodukte Emulphor EL-620 der Firma Rhone Poulenc, Paris, Frankreich, Emulphor EL-719 (Rhone Poulenc) und Cremophor EL der Firma BASF, Ludwigshafen, Deutschland verwendet werden. Das, bevorzugt mit 40 Mol Ethylenoxid oxethylierte, hydrierte Rizinusöl, kann beispielsweise in Form der Handelsprodukte Cremophor RH 40 und Cremophor RH 410 (BASF) verwendet werden. Als Polyoxyethylensorbitanmonofettsäureester, kann beispielsweise das mit 20 Mol Ethylenoxid oxethylierte Sorbitanmonopalmitat, zum Beispiel in Form des Handelsprodukts Tween 40 der Firma ICI Americas, Wilmington, USA, oder das mit 20 Mol Ethylenoxid oxethylierte Sorbitanmonostearat in Form des Handelsproduktes Tween 60 (ICI Americas), verwendet werden. Als anionische Tenside werden bevorzugt Alkali-, Erdalkali-, Ammonium- oder Alkanolminsalze von Alkansulfonaten, Alkylbenzolsulfonaten, Alkylethersulfaten, Alkylethercarboxylaten und Alkylsulfaten, wie beispielsweise C₁₂-bis C₁₈-Alkylsulfatnatriumsalze, insbesondere Natriumcetylstearylsulfat und Natriumlaurylsulfat, verwendet.

Das Haarbehandlungsmittel enthält die Komponente (D) bevorzugt in einer Menge von 25 bis 35 Gewichtsprozent.

Das Haarbehandlungsmittel gemäß der vorliegenden Erfindung kann zusätzlich alle diejenigen Bestandteile enthalten, die in Haarbehandlungsmitteln üblicherweise eingesetzt werden, insbesondere kationische, amphotere oder zwitterionische Tenside, beispielsweise oxethylierte Fettalkohole, Fettsäurealkanolamide, Alkyltrimethylammoniumsalze, Alkylbetaine, Alkylaminobetaine, Alkylsulfobetaine und Fettsäurealkylamidobetaine in einer Menge von 0,01 bis bis 50 Gewichtsprozent; Schaumsynergisten; Schaumstabilisatoren Sequestriermittel; Emulgatoren; Naturstoffe; Pigmente; Parfümöle in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,5 bis 10,0 Gewichtsprozent; Puffersubstanzen, wie beispielsweise Natriumcitrat oder Natriumphosphat, in einer Menge von 0,1 bis 1,0 Gewichtsprozent; sowie Farbstoffe, wie zum Beispiel Fluorescein-Natriumsalz, Gelb ZN3 (C.I. 47 055), in einer Menge von 0,1 bis 1,0 Gewichtsprozent; weiterhin haarpflegende Zusätze, wie zum Beispiel Fettsäureester, Fettalkohole, Fettsäureglyceride, ethoxylierte oder propoxylierte gesättigte Fettalkohole; natürliche, modifizierte natürliche oder synthetische Polymere, wie zum Beispiel kationische, anionische oder nichtionische Cellulosederivate, Chitosan, kationische Chitin- oder Chitosanderivate, Polyvinylpyrrolidon oder Polymerisate von Acrylsäurederivaten; Pflegestoffe, wie zum Beispiel Lanolinderivate, Cholesterin und Pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Feuchthaltemittel; Lichtschutzmittel; Antioxidantien; Komplexbildner; Antischuppenwirkstoffe; kosmetische Öle und Wachse sowie Konservierungsstoffe, soweit solche Zusätze nützlich und zweckmäßig erscheinen und mit den Bestandteilen des erfindungsgemäßen Mittels verträglich sind.

Das erfindungsgemäße Mittel wird bevorzugt als Dauerwellvorbehandlungsmittel, das heißt zur Behandlung des Haares vor dem Wickeln auf Dauerwellwickler, verwendet.

Die vorliegende Erfindung betrifft daher weiterhin ein Verfahren zur dauerhaften Verformung von Haaren, bei dem man das Haar vor dem Wickeln auf Dauerwellwickler mit einem Dauerwellvorbehandlungsmittel behandelt, auf Wickler wickelt, sodann mit einem Haardauerverformungsmittel behandelt, nach einer für die dauerhafte Verformung des Haares ausreichenden Einwirkungszeit mit Wasser spült, sodann oxidativ nachbehandelt, mit Wasser spült, anschließend zur Frisur legt und sodann trocknet, welches dadurch gekennzeichnet ist, daß als Dauerwellvorbehandlungsmittel das vorstehend beschriebene Mittel verwendet wird.

Bei dem erfindungsgemäßen Verfahren wird das Haar zunächst mit einem Shampoo gewaschen und danach mit Wasser gespült. Anschließend wird eine für die Dauerwellvorbehandlung ausreichende Menge, vorzugsweise etwa 3 bis 6 g, des Dauerwellvorbehandlungsmittels auf das handtuchtrockene Haar, vorzugsweise auf die Haarspitzen, aufgetragen, das Haar in Strähnen aufgeteilt und auf Dauerwellwickler gewickelt. Der Durchmesser der Wickler beträgt hierbei etwa 5 bis 35 Millimeter. Sodann wird das Haar mit einer für die dauerhafte Haarverformung ausreichenden Menge, vorzugsweise etwa 60 bis 90 g, eines Haardauerverformungsmittels behandelt.

Bei dem Haardauerverformungsmittel handelt es sich insbesondere um eine wäßrige, alkalisch (pH = 7 bis 10) eingestellte Zubereitung, welche eine keratinreduzierende Mercaptoverbindung, wie zum Beispiel Cystein, Cysteamin, N-Acetyl-L-Cystein, Mercaptocarbonsäuren, beispielsweise Thioglykolsäure oder Thiomilchsäure, oder Salze von Mercaptocarbonsäuren, wie zum Beispiel Ammonium- und Guanidinsalze der Thioglykolsäure oder Thiomilchsäure, in einer Konzentration von etwa 2 bis 12 Gewichtsprozent enthält.

Die erforderliche Alkalität wird hierbei durch die Zugabe von Ammoniak, organischen Aminen, Ammonium- und Alkalicarbonate oder -hydrogencarbonat eingestellt. Es kommt aber auch ein neutral oder sauer (pH = 4,5 bis 7) eingestelltes Haarverformungsmittel in Betracht, das in wäßrigem Medium einen wirksamen Gehalt an Sulfiten oder Mercaptocarbonsäureestern aufweist.

Im ersteren Fall werden vorzugsweise Natrium- oder Ammoniumsulfit oder das Salz der schwefligen Säure mit einem organischen Amin, wie zum Beispiel Monoethanolamin und Guanidin, in einer Konzentration von etwa 2 bis 12 Gewichtsprozent (berechnet als SO₂) verwendet. Im letzteren Fall kommen insbesondere Thioglykolsäuremonoglykolester oder -glycerinester in einer Konzentration von etwa 5 bis 50 Gewichtsprozent (entsprechend einem Gehalt an freier Thioglykolsäure von 2 bis 16 Gewichtsprozent) zur Anwendung.

Das Mittel zur dauerhaften Haarverformung kann auch ein Gemisch der vorstehend genannten keratinreduzierenden Verbindungen enthalten.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur etwa 10 bis 30 Minuten beträgt, wird das Haar mit Wasser gespült und anschließend oxidativ nachbehandelt ("fixiert"). Das Nachbehandlungsmittel wird, je nach Haarfülle, in einer Menge von etwa 50 bis 100 g verwendet.

Für die oxidative Nachbehandlung kann jedes beliebige, bisher für eine derartige Behandlung verwendete, Nachbehandlungsmittel verwendet werden. Beispiele für in einem derartigen Nachbehandlungsmittel verwendbare Oxidationsmittel sind Natrium- und Kaliumbromat, Natriumperborat, Harnstoffperoxid und Hydrogenperoxid.

Die Konzentration des Oxidationsmittels ist in Abhängigkeit von der Anwendungszeit (in der Regel etwa 5 bis 15 Minuten) und der Anwendungstemperatur unterschiedlich. Üblicherweise liegt das Oxidationsmittel in dem wäßrigen Nachbehandlungsmittel in einer Konzentration von etwa 0,5 bis 10 Gewichtsprozent vor.

Sowohl das Mittel zur dauerhaften Haarverformung als auch das Mittel zur oxidativen Nachbehandlung kann in Form einer wäßrigen Lösung oder Emulsion sowie in verdickter Form auf wäßriger Basis, insbesondere als Creme, Gel oder Paste, vorliegen. Ebenfalls ist es möglich, dieses Mittel unter Druck in Aerosoldosen abzufüllen und daraus als Schaum zu entnehmen.

Anschließend werden die Wickler entfernt. Falls erforderlich, kann das abgewickelte Haar nun nochmals oxidativ nachbehandelt werden. Sodann wird das Haar mit Wasser gespült, zur Frisur gelegt und schließlich getrocknet.

Das vorstehend beschriebene erfindungsgemäße Verfahren zur dauerhaften Haarverformung ermöglicht eine schonende und gleichmäßige Umformung vom Haaransatz bis zu den Haarspitzen, das Haar zeigt eine hervorragende Naß- und Trockkämmbarkeit, einen angenehmen Griff und einen ansprechenden Glanz im getrockneten Zustand, sowie eine lockere, sprunghafte und gleichmäßige Dauerwellung, insbesondere im Bereich der Haarspitzen.

Ein weiterer Gegenstand der vorliegenden Anmeldung ist ein, auch ohne nachfolgende Dauerwellung anzuwendendes, Verfahren zur Behandlung von Haaren, dadurch gekennzeichnet, daß man, nach der Haarwäsche mit nicht-konditionierenden Shampoo, das Haarbehandlungsmittel, je nach Haarfülle, in einer Menge von 5 bis 15 g, in dem getrockneten Haar verteilt und sodann das Haar zur Frisur kämmt.

Das erfindungsgemäße Mittel läßt sich gut im getrockneten Haar verteilen.

Die mit dem erfindungsgemäßen Mittel behandelten Haare zeigen eine hervorragende Naß- und Trockenkämmbarkeit, einen angenehmen Griff und einen ansprechenden Glanz im getrockneten Zustand.

Folgende Beispiele sollen den Gegenstand der Erfindung näher erläutern:

### Beispiele

### Beispiel 1: Haarbehandlungsmittel in Gelform

- 1,00 g: Acrylsäurehomopolymer mit einem Molekulargewicht von 3.000.000
- 15,00 g: Dimethylpolysiloxan mit einer Viskosität von 500 mm²·s⁻¹
- 0,27 g: mit 40 Mol Ethylenoxid oxethyliertes hydriertes Rizinusöl
- 28,41 g: Ethanol
- 0,88 g: Ammoniak, 25 prozentige wäßrige Lösung
- 54,44 g: Wasser, vollentsalzt
- 100,00 g:

Der pH-Wert wird mit wäßriger Ammoniaklösung auf 7 eingestellt.

### Beispiel 2: Haarbehandlungsmittel in Gelform

- 1,20 g: Acrylsäurehomopolymer mit einem Molekulargewicht von 3.000.000
- 20,00 g: Dimethylpolysiloxan mit einer Viskosität von 500 mm²·s⁻¹
- 0,49 g: mit 20 Mol Ethylenoxid oxethyliertes Sorbitanmonopalmitat
- 23,68 g: Ethanol
- 1,06 g: Ammoniak, 25 prozentige wäßrige Lösung
- 53,57 g: Wasser, vollentsalzt
- 100,00 g:

Der pH-Wert wird mit wäßriger Ammoniaklösung auf 7 eingestellt.

### Beispiel 3: Haarbehandlungsmittel in Gelform

- 1,40 g: Acrylsäurehomopolymer mit einem Molekulargewicht von 4.000.000
- 18,00 g: Dimethylpolysiloxan mit einer Viskosität von 500 mm²·s⁻¹
- 0,39 g: mit 20 Mol Ethylenoxid oxethyliertes Sorbitanmonopalmitat
- 35,00 g: Isopropanol
- 1,23 g: Ammoniak, 25 prozentige wäßrige Lösung
- 43,98 g: Wasser, vollentsalzt
- 100,00 g:

Der pH-Wert wird mit wäßriger Ammoniaklösung auf 7 eingestellt.

### Beispiel 4: Haarbehandlungsmittel in Gelform

- 0,400 g: Acrylsäurehomopolymer mit einem Molekulargewicht von 4.000.000
- 30,000 g: Dimethylpolysiloxan mit einer Viskosität von 1.000 mm²·s⁻¹
- 0,450 g: mit 40 Mol Ethylenoxid oxethyliertes Rizinusöl
- 33,145 g: Ethanol
- 0,400 g: Parfümöl
- 1,000 g: Kamillenblütenextrakt
- 0,001 g: Gelb ZN3 (C.I. 47 055)
- 0,352 g: Ammoniak, 25 prozentige wäßrige Lösung
- 34,252 g: Wasser, vollentsalzt
- 100,000 g:

Der pH-Wert wird mit wäßriger Ammoniaklösung auf 7 eingestellt.

### Beispiel 5: Haarbehandlungsmittel in Gelform

- 1,00 g: Acrylsäurehomopolymer mit einem Molekulargewicht von 3.000.000
- 15,00 g: Dimethylpolysiloxan mit einer Viskosität von 500 mm²·s⁻¹
- 0,27 g: mit 40 Mol Ethylenoxid oxethyliertes hydriertes Rizinusöl
- 30,00 g: Isopropanol
- 0,10 g: Polyhexamethylen-biguanid-hydrochlorid, 20 prozentige wäßrige Lösung
- 0,88 g: Ammoniak, 25 prozentige wäßrige Lösung
- 47,75 g: Wasser, vollentsalzt
- 100,00 g:

Der pH-Wert wird mit wäßriger Ammoniaklösung auf 7 eingestellt.

### Beispiel 6: Haarbehandlungsmittel in Gelform

- 1,00 g: Acrylsäurehomopolymer mit einem Molekulargewicht von 3.000.000
- 15,00 g: Dimethylpolysiloxan mit einer Viskosität von 500 mm²·s⁻¹
- 0,15 g: Natriumcetylstearylsulfat
- 28,41 g: Ethanol
- 0,88 g: Ammoniak, 25 prozentige wäßrige Lösung
- 54,56 g: Wasser, vollentsalzt
- 100,00 g:

Der pH-Wert wird mit wäßriger Ammoniaklösung auf 7 eingestellt.

### Beispiel 7: Haarbehandlungsmittel in Gelform

- 1,40 g: Acrylsäurehomopolymer mit einem Molekulargewicht von 4.000.000
- 16,00 g: Dimethylpolysiloxan mit einer Viskosität von 500 mm²·s⁻¹
- 0,50 g: Natriumlaurylpolyglykolethersulfat, 28 prozentige wäßrige Lösung
- 23,68 g: Ethanol
- 1,23 g: Ammoniak, 25 prozentige wäßrige Lösung
- 57,19 g: Wasser, vollentsalzt
- 100,00 g:

Der pH-Wert wird mit wäßriger Ammoniaklösung auf 7 eingestellt.

### Vergleichsbeispiele

In den Vergleichsbeispielen A, B und C werden die Eigenschaften der erfindungsgemäßen Mittel als Dauerwellvorbehandlungsmittel mit den Eigenschaften von aus dem Stand der Technik bekannten Präparaten ähnlicher Zusammensetzung verglichen.

### Vergleichsbeispiel A:

Nach einer vorangegangenen Haarwäsche mit einem üblichen, nicht konditionierenden Shampoo wurde das handtuchtrockene Haar von 30 Versuchspersonen mit einem Haarbehandlungsmittel gemäß Beispiel 1 behandelt. Von den Versuchspersonen hatten 10 Personen an den Haarspitzen strapaziertes Haar, 10 Personen gesträhnt blondiertes Haar und 10 Personen dauergewelltes Haar, das am Haaransatz glatt nachgewachsen war. Um eindeutige Ergebnisse zu erhalten und von Versuchsperson zu Versuchsperson abweichende Haarqualitäten auszuschalten, wurde das Haar in der Mitte gescheitelt und auf der einen Hälfte der Haare, insbesondere im Bereich der Haarspitzen oder Strähnen, in Abhängigkeit von der Haarfülle, 1,5 bis 3 g des erfindungsgemäßen Mittels verteilt, während die andere Hälfte mit der gleichen Menge des nachstehenden Vergleichspräparats I, gemäß der im Stand der Technik zitierten US-PS 4 954 335, behandelt wurde. Nach dem Durchkämmen und Aufwickeln des Haares auf Dauerwellwickler, wurde in üblicher Weise eine Dauerwellbehandlung durchgeführt. So konnte die Wirkung der Präparate von einer friseurfachlichen Expertengruppe sicher beurteilt werden. Eine Bewertung erfolgte nach dem Schema für die zusammengefaßten Kriterien der Naß- und Trockenkämmbarkeit, Gleichmäßigkeit und Sprunghaftigkeit der Dauerwelle sowie Lockigkeit, Griff und Glanz des Haares, insbesondere im Bereich der Haarspitzen.

### Benotung:

1 = sehr gut
2 = gut
3 = ausreichend
4 = mangelhaft

### Vergleichspräparat I (gemäß US-PS 4 954 335)

- 0,75 g: Hydroxyethylcellulose
- 1,00 g: flüchtiges Cyclomethicon
- 2,00 g: N-Dodecyl-2-pyrrolidon
- 0,80 g: Stearamidopropyldimethylamin
- 0,30 g: Milchsäure
- 2,00 g: Dicetyldiammoniumchlorid
- 1,20 g: mit 10 Mol Ethylenoxid oxethyliertes Isooctylphenol
- 9,00 g: Ethanol
- 0,15 g: Konservierungsmittel
- 0,40 g: Parfümöl
- 82,40 g: Wasser, vollensalzt
- 100,00 g:

Das Ergebnis des Vergleichs beider Mittel zeigt Tabelle 1.

**Tabelle 1**

| | Note 1 | Note 2 | Note 3 | Note 4 |
|---|---|---|---|---|
| Mittel gemäß Beispiel 1 (Anzahl der Versuchspersonen) | 13 | 17 | 0 | 0 |
| Vergleichspräparat I (Anzahl der Versuchspersonen) | 0 | 6 | 16 | 8 |

### Vergleichsbeispiel B:

Die Haare einer weiteren Gruppe von 32 Versuchspersonen, von denen 12 Personen an den Haarsptizen stark stapaziertes Haar, 10 Personen gesträhnt blondiertes Haar und 10 Personen dauergewelltes Haar, das am Haaransatz glatt nachgewachsen war, hatten, wurden mit einem Haarbehandlungsmittel gemäß Beispiel 1 und den nachstehenden Vergleichspräparaten II, III, IV und V, entsprechend der in Stand der Technik zitierten EP-OS 0 260 641, in gleicher Weise wie in Vergleichsbeispiel A mit je 1,5 bis 3 g der Mittel behandelt.

### Vergleichspräparat II (entsprechend EP-OS 0 260 641)

- 2,00 g: Vinylacetat/Crotonsäure-Copolymer
- 0,20 g: 2-Amino-2-methyl-1,3-propandiol
- 0,50 g: Dimethylpolysiloxan/Polyoxyalkylen-Copolymer
- 0,50 g: mit 20 Mol Ethylenoxid oxethyliertes Sorbitanmonooleat
- 0,10 g: Stearyltrimethylammoniumchlorid
- 1,50 g: Dimethylpolysiloxan mit einer Viskosität von 100 mm².s⁻¹
- 1,50 g: Methylphenylpolysiloxan mit einer Viskosität von 300 mm².s⁻¹
- 10,00 g: Ethanol
- 10,00 g: Treibmittel
- 73,70 g: Wasser
- 100,00 g:

### Vergleichspräparat III (entsprechend EP-OS 0 260 641)

- 2,00 g: Vinylacetat/Crotonsäure-Copolymer
- 0,20 g: 2-Amino-2-methyl-1,3-propandiol
- 0,50 g: Dimethylpolysiloxan/Polyoxyalkylen-Copolymer
- 0,40 g: mit 20 Mol Ethylenoxid oxethyliertes Sorbitanmonooleat
- 0,08 g: Stearyltrimethylammoniumchlorid
- 2,00 g: Dimethylpolysiloxan mit einer Viskosität von 100 mm².s⁻¹
- 20,00 g: Ethanol
- 10,00 g: Treibmittel
- 64,82 g: Wasser
- 100,00 g:

### Vergleichspräparat IV (entsprechend EP-OS 0 260 641)

- 2,00 g: Vinylacetat/Crotonsäure-Copolymer
- 0,20 g: 2-Amino-2-methyl-1,3-propandiol
- 0,93 g: Dimethylpolysiloxan/Polyoxyalkylen-Copolymer
- 0,12 g: mit 20 Mol Ethylenoxid oxethyliertes Sorbitanmonooleat
- 0,05 g: Stearyltrimethylammoniumchlorid
- 1,50 g: Dimethylpolysiloxan mit einer Viskosität von 100 mm².s⁻¹
- 1,50 g: Methylphenylpolysiloxan mit einer Viskosität von 300 mm².s⁻¹
- 20,00 g: Ethanol
- 10,00 g: Treibmittel
- 73,70 g: Wasser
- 100,00 g:

### Vergleichspräparat V (entsprechend EP-OS 0 260 641)

- 2,00 g: Vinylacetat/Crotonsäure-Copolymer
- 0,20 g: 2-Amino-2-methyl-1,3-propandiol
- 0,50 g: Dimethylpolysiloxan/Polyoxyalkylen-Copolymer
- 0,50 g: mit 20 Mol Ethylenoxid oxethyliertes Sorbitanmonooleat
- 0,10 g: Stearyltrimethylammoniumchlorid
- 1,50 g: Methylphenylpolysiloxan mit einer Viskosität von 1.000 mm².s⁻¹
- 10,00 g: Ethanol
- 10,00 g: Treibmittel
- 75,20 g: Wasser
- 100,00 g:

Das Ergebnis des Vergleichs des erfindungsgemäßen Mittels mit den Vergleichspräparaten II, III, IV und V zeigt Tabelle 2.

**Tabelle 2**

| | Note 1 | Note 2 | Note 3 | Note 4 |
|---|---|---|---|---|
| Mittel gemäß Beispiel 1 (Anzahl der Versuchspersonen) | 19 | 12 | 1 | 0 |
| Vergleichspräparate II, III, IV und V (Anzahl der Versuchspersonen) | 0 | 5 | 24 | 3 |

### Vergleichsbeispiel C:

Die Haare einer weiteren Gruppe von 28 Versuchspersonen von denen 10 Personen an den Haarspitzen stark strapaziertes Haar, 9 Personen gesträhnt blondiertes und 9 Personen dauergewelltes Haar, das am Haaransatz glatt nachgewachsen war, hatten, wurden mit einem Haarbehandlungsmittel gemäß Beispiel 1 und dem nachstehenden Vergleichspräparat VI, entsprechend der in Stand der Technik zitierten PCT/WO 89/04164, in gleicherweise wie in Vergleichsbeispiel A mit je 1,5 bis 3 g der Mittel behandelt.
Vergleichspräparat VI (entsprechend PCT/WO 89/04164)
- 2,00 g: Hydroxyethylcellulose
- 2,00 g: Dimethylpolysiloxan mit einer Viskosität von 500 mm²·s⁻¹
- 2,00 g: mit 40 Mol Ethylenoxid oxethyliertes hydriertes Rizinusöl
- 2,50 g: Cetyltrimethylammoniumchlorid, 50 %ige wäßrige Lösung
- 2,50 g: Polyethylenglykol, aus 4 Ethylenoxideinheiten
- 0,30 g: Parfümöl
- 88,70 g: Wasser, vollentsalzt
- 100,00 g:

Das Ergebnis des Vergleichs des erfindungsgemäßen Mittels mit dem Vergleichspräparat VI zeigt Tabelle 3.

**Tabelle 3**

| | Note 1 | Note 2 | Note 3 | Note 4 |
|---|---|---|---|---|
| Mittel gemäß Beispiel 1 (Anzahl der Versuchspersonen) | 19 | 9 | 0 | 0 |
| Vergleichspräparat VI (Anzahl der Versuchspersonen) | 0 | 7 | 10 | 11 |

Die Vergleichsbeispiele A bis C zeigen die hervorragende schützende und pflegende Wirkung des erfindungsgemäßen Mittels gegenüber den Vergleichspräparaten I bis VI ähnlicher Zusammensetzung, bei der Anwendung als Dauerwellvorbehandlungsmittel. Das Haar zeigt, trotz unterschiedlicher Qualität, eine gleichmäßige und sprunghafte Dauerwellung bei sehr guter Naß- und Trockenkämmbarkeit. Im trockenen Zustand hat das Haar einen ansprechenden Griff und Glanz.

### Vergleichsbeispiele D bis F

In den Vergleichsbeispielen D, E und F werden die erfindungsgemäßen Mittel mit den aus dem Stand der Technik bekannten Präparaten ähnlicher Zusammensetzung (Vergleichspräparate I bis VI) bezüglich ihrer Eigenschaften als Haarglanzmittel verglichen.

### Vergleichsbeispiel D:

Nach vorangegangener Haarwäsche mit einem üblichen nicht konditionierenden Shampoo wurde das getrocknete stark strapazierte Haar von 30 Versuchspersonen mit einem Haarbehandlungsmittel gemäß Beispiel 1 behandelt. Das Haar wurde in der Mitte gescheitelt und auf der einen Hälfte des Haares, in Abhängigkeit von der Haarfülle, 2,5 bis 7,5 g des erfindungsgemäßen Mittels aufgetragen und im Haar verteilt, während die andere Hälfte mit der gleichen Menges des Vergleichspräparates I behandelt wurde.

Eine Bewertung erfolgte durch eine friseur-fachliche Expertengruppe nach dem Schema für die zusammengefaßten Kriterien der Naß- und Trockenkämmbarkeit, Griff und Glanz des Haares.

Das Ergebnis des Vergleichs des erfindungsgemäßen Mittels mit dem Vergleichspräparat I zeigt Tabelle 4.

**Tabelle 4**

| | Note 1 | Note 2 | Note 3 | Note 4 |
|---|---|---|---|---|
| Mittel gemäß Beispiel 1 (Anzahl der Versuchspersonen) | 26 | 4 | 0 | 0 |
| Vergleichspräparat I (Anzahl der Versuchspersonen) | 0 | 20 | 10 | 0 |

### Vergleichsbeispiel E:

Die Haare einer weiteren Gruppe von 32 Versuchspersonen mit stark strapaziertem Haar wurden mit einem Haarbehandlungsmittel gemäß Beispiel 1 und den Vergleichspräparaten II, III, IV und V in gleicher Weise wie in Vergleichsbeispiel D mit je 2,5 bis 7,5 g der Mittel behandelt.

Das Ergebnis des Vergleichs des erfindungsgemäßen Mittels mit den Vergleichspräparaten II, III, IV und V zeigt Tabelle 5.

**Tabelle 5**

| | Note 1 | Note 2 | Note 3 | Note 4 |
|---|---|---|---|---|
| Mittel gemäß Beispiel 1 (Anzahl der Versuchspersonen) | 23 | 9 | 0 | 0 |
| Vergleichspräparate II, III, IV und V (Anzahl der Versuchspersonen) | 0 | 19 | 13 | 0 |

### Vergleichsbeispiel F:

Die Haare einer weiteren Gruppe von 28 Versuchspersonen mit stark strapaziertem Haar wurden mit einem Haarbehandlungsmittel gemäß Beispiel 1 und Vergleichspräparat VI in gleicherweise wie in Vergleichsbeispiel D mit je 2,5 bis 7,5 g der Mittel behandelt.

Das Ergebnis des Vergleichs des erfindungsgemäßen Mittels mit dem Vergleichspräparat III zeigt Tabelle 6.

**Tabelle 6**

| | Note 1 | Note 2 | Note 3 | Note 4 |
|---|---|---|---|---|
| Mittel gemäß Beispiel 1 (Anzahl der Versuchspersonen) | 22 | 6 | 0 | 0 |
| Vergleichspräparat III (Anzahl der Versuchspersonen) | 0 | 11 | 17 | 0 |

Die Vergleichsbeispiele D bis F zeigen die hervorragende glanzgebende Wirkung des erfindungsgemäßen Mittels gegenüber Mitteln ähnlicher Zusammensetzung.

Sämtliche in der Anmeldung angegebenen Prozentzahlen stellen Gewichtsprozente dar.

## Patentansprüche

1. Gelförmiges Dauerwellvorbehandlungsmittel, dadurch gekennzeichnet, daß es
(A) neben der Komponente (B) als einziges weiteres Polymer 0,3 bis 4 Gewichtsprozent mindestens eines hochmolekularen Acrylsäurehomopolymers mit einem Molekulargewicht von 1.000.000 bis 4.000.000,
(B) 15 bis 30 Gewichtsprozent mindestes eines nicht-flüchtigen Polydimethylsiloxans,
(C) 0,1 bis 0,5 Gewichtsprozent mindestens eines nicht-ionischen und/oder anionischen Tensids und
(D) 10 bis 40 Gewichtsprozent Ethanol und/oder Isopropanol
enthält.

2. Mittel nach Anspruch 1, dadurch gekennzeichnet, daß es 0,4 bis 2 Gewichtsprozent der Komponente (A) enthält.

3. Mittel nach Anspruch 1, dadurch gekennzeichnet, daß es 25 bis 35 Gewichtsprozent der Komponente (D) enthält.

4. Verfahren zur dauerhaften Verformung von Haaren, bei dem man das Haar, insbesondere die Haarspitzen, vor dem Wickeln auf Dauerwellwickler mit einem Dauerwellvorbehandlungsmittel behandelt, auf Wickler wickelt, sodann mit einem Haardauerverformungsmittel behandelt, nach einer für die dauerhafte Verformung des Haares ausreichenden Einwirkungszeit mit Wasser spült, sodann oxidativ nachbehandelt, mit Wasser spült, anschließend zur Frisur legt und sodann trocknet, dadurch gekennzeichent, daß als Dauerwellvorbehandlungsmittel ein Mittel nach einem der Ansprüche 1 bis 3 verwendet wird.

5. Verfahren nach Anspruch 4, dadurch gekennzeichnet, daß man das Dauerwellvorbehandlungsmittel in einer Menge von 3 bis 6 g anwendet.

## Claims

1. Permanent waving pretreatment agent in gel form, characterised in that it contains:
(A) in addition to component (B) as the sole further polymer from 0.3 to 4 weight percent of at least one high-molecular acrylic acid homopolymer with a molecular weight of from 1,000,000 to 4,000,000;
(B) 15 to 30 weight percent of at least one non-volatile polydimethylsiloxane;
(C) from 0.1 to 0.5 weight percent of at least one non-tonic and/or anionic surfactant; and
(D) from 10 to 40 weight percent of ethanol and/or isopropanol.

2. Agent according to Claim 1, characterised in that it contains from 0.4 to 2 weight percent of component (A).

3. Agent according to Claim 1, characterised in that it contains from 25 to 35 weight percent of component (D).

4. Process for permanently shaping hair in which the hair, in particular the tips of the hair, before being wound onto permanent waving rollers is treated with a permanent waving pretreatment agent, is wound onto rollers, is then treated with a permanent hair shaping agent, is washed with water after a reaction time sufficient for the permanent shaping of the hair, is then post-treated oxidatively, rinsed with water, subsequently set in a hairstyle and then dried, characterised in that an agent according to any one of Claims 1 to 3 is used as the permanent waving pretreatment agent.

5. Process according to Claim 4, characterised in that the permanent waving pretreatment agent is used in an amount of from 3 to 6 g.

## Revendications

1. Agent géliforme de mise en plis permanente caractérisé par le fait qu'il comporte :
(A) comme seul polymère autre que le composant (B), 0,3 à 4% en poids d'au moins d'un homopolymère de l'acide acrylique de masse moléculaire élevée, avec une masse moléculaire de 1 000 000 à 4 000 000,
(B) de 15 à 30% en poids d'au moins un polydiméthylsiloxane non volatil,
(C) de 0,1 à 0,5% en poids d'un agent tensio-actif non-ionogène et/ou anionique,
(D) de 10 à 40% en poids d'éthanol et/ou d'isopropanol.

2. Agent selon la revendication 1, caractérisé en ce qu'il contient de 0,4 à 2% en poids du composant (A).

3. Agent selon la revendication 1, caractérisé en ce qu'il contient de 25 à 35% en poids du composant (D).

4. Procédé pour mise en pli permanente des cheveux au cours de laquelle on traite les cheveux, notamment les pointes, avant l'enroulement sur les rouleaux avec un agent de pré-traitement de permanente, on les enroule sur les rouleaux, puis on les traite avec un agent de mise en pli permanente, on les rince à l'eau après une mise en pli durable des cheveux pendant un temps d'action suffisant, puis on opère un post-traitement oxydant, enfin, on les coiffe et on les sèche, caractérisé en ce que comme agent de mise en pli permanente, on utilise un agent selon l'une des revendications 1 à 3.

5. Procédé selon la revendication 4, caractérisé en ce que l'on utilise l'agent de mise en pli permanente à raison de 3 à 6 g.
